# EUROPEAN PATENT APPLICATION

(11) **EP 3 109 317 A1**
(43) Date of publication of application: **28.12.2016**
(21) Application number: 16178501.9
(22) Date of filing: 17.10.2008
(51) Int. Cl.: C12N 5/0775, C12N 5/0783, A61K 39/00

(54) **TR1 CELLS, MESENCHYMAL STEM CELLS AND USES THEREOF**

(30) Priority: 17.10.2007 US 980541 P
(62) Divisional of application: 08840358.9
(71) Applicant: TXCell, 06560 Valbonne (FR)
(72) Inventor: FOUSSAT, Arnaud, 06560 Valbonne (FR); BELMONTE, Nathalie, 06200 Nice (FR)
(74) Representative: Icosa

(57) **Abstract**

The present invention relates to compositions comprising Tr1 cells and mesenchymal stem cells and methods for treating an autoimmune disease, an allergic disease or an inflammatory disease.

## Description

### FIELD OF THE INVENTION

This invention relates to Tr1 cells and mesenchymal stem cells. More particularly, this invention relates to uses of Tr1 cells and mesenchymal stem cells for treating an excessive, dysfunctional or uncontrolled self or non self T cell mediated response, such as autoimmune disease or inflammatory disease.

### BACKGROUND OF THE INVENTION

The function of the immune system is to eliminate foreign cells that may contain pathogens, while maintaining unresponsiveness or tolerance against self-antigens. Tolerance is manifested by autoreactive-T cell depletion or anergy, the latter being characterized by the survival, but hyporesponsiveness of T cells. However, in several circumstances the immune system may attack self-constituents, causing autoimmune disease. Autoimmune diseases are believed to originate in the abnormal immune response to self-antigens, either due to a change in self-antigens immunogenic capacity or exposure to cross-reactive mimetic antigens.

It is desirable to improve present treatments of autoimmune disease or other undesirable immune reactions which use general immunosuppressive agents such as anti-TNF compounds, corticosteroids, azathioprine, or cyclosporine A. These treatments are nonselective and do not distinguish between normal and abnormal immune responses. These drugs often have adverse side effects, including general suppression of the immune system with high risks of infection and neoplasia, as well as the development of diseases such as diabetes, osteoporosis, leukopenia and hypertension. Alternative approaches for treatment of these conditions are needed for patients who cannot withstand, or do not respond to, conventional nonspecific drug therapy. These alternative approaches are based on the induction of immunosuppression and/or specific immune tolerance, aimed at "silencing" the pathogenic response to self-antigen, while keeping host defense mechanisms intact.

Alternative approaches using MSCs have been considered.

Mesenchymal stem cells (MSCs) are multipotent stem cells that can readily differentiate into lineages including osteoblasts, myocytes, chondrocytes and adipocytes. MSCs express major histocompatibility complex (MHC) class I antigen on their surface but limited class II and no B7 or CD40 co-stimulatory molecules, suggesting that these cells have a low immunogenic capacity. MSCs also inhibit T-cell proliferative responses in an MHC-independent manner. These immunological properties of MSCs may enhance their transplant engraftment and limit the ability of the recipient immune system to recognize and reject allogeneic cells following transplantation.

The patent application US2002/044923 discloses the use of MSCs, which have been modified to carry an antigen, to treat or inhibit an unwanted or abnormal immune response such as it occurs in autoimmune disease. Presentation of the antigen to the T cell in the absence of a costimulatory signal induces an antigen-specific state of hyporesponsiveness, or even nonresponsiveness or anergy in the T cell to subsequent challenge of the T cell by the antigen.

The patent application WO2005/093044 (Pittenger et al.) describes how MSCs may be employed in the treatment of disease conditions and disorders involving the immune system. Pittenger et al. believe that 1/ MSCs can stimulate dendritic cells (DCs) to produce Interferon-beta (IFN-β), which promotes tumor suppression and immunity against viral infection, and 2/ MSCs can suppress autoimmune disease by causing the release of interleukin-10 (IL-10) from regulatory T-cells (Treg cells) and/or DC. However, all experiments shown in the examples are realized in vitro and Pittenger et al. do not demonstrate in this patent application that injection of MSCs can effectively treat disease conditions and disorders involving the immune system.

The patent application US2002/085996 relates to the use of MSCs to prevent, reduce or treat transplant rejection and/or graft versus host reaction, but is silent regarding autoimmune conditions.

Other alternative approaches using regulatory T cells have been considered.

Several subsets of regulatory T (Treg) cells with distinct phenotypes and distinct mechanisms of action have now been identified. These include CD4+CD25+ Treg cells, which inhibit immune responses through cell-cell contact, Th3 cells which primarily secrete TGF-β, and Tr1 cells which secrete high levels of IL-10 and low to moderate levels of TGF-β. These Tr1 cells produce IL-10, IL-5 and IFN-γ, with or without TGF-β, but with little or no IL-2 or IL-4, and proliferate poorly following polyclonal TCR-mediated activation.

The patent applications US2007/009497 and WO2006/090291 disclose the use of CD4+CD25+ Treg cells for treating autoimmune and inflammatory conditions.

The patent US6281012 discloses the use of suppressor T cells to reduce or inhibit host rejection of the transplant. These suppressor T cells are defined as T cells which have been primed in a mixed lymphocyte reaction by exposure to an alloantigen, and subsequently cultured with mesenchymal stem cells. These suppressor T cells therefore comprise allogeneic Tr1 cells.

Trough, the patent application WO2006/018674, the applicants discloses the use of Tr1 cells to treat atherosclerosis. The Applicants also observed in a mice model of Crohn's disease wherein pro-inflammatory cells are directed against commensal bacteria of the digestive flora, that administration to mice of Tr1 cells directed against a antigen delivered in food allow the prevention of chronic inflammation of colon.

The Applicants aim now to provide a new alternative treatment to improve existing treatments of autoimmune disease or other undesirable immune reactions. This new treatment is still based on the induction of immunosuppression and/or specific immune tolerance, aimed at "silencing" the pathogenic response to self-antigen, while keeping host defense mechanisms intact. This new treatment is based on the use of a composition comprising Tr1 cells and mesenchymal stem cells. The Applicants surprisingly found out that treatment with a composition comprising Tr1 cells and mesenchymal stem cells give better results than treatment with only MSCs or Tr1 cells. Without wishing to be bound to a theory, the Applicants believe that this composition may induce antigen specific immune tolerance in a subject to treat an excessive, dysfunctional or uncontrolled self or non self T cell mediated immune response, via different pathways such as:
1. inhibition of T-cell proliferative responses by MSCs in an antigen-independent manner;
2. inhibition of T-cell proliferative responses and induction of T cell tolerance by IL-10 secreted by Tr1 cells;
3. in vivo induction of Tr1 cells by MSCs.

### SUMMARY OF THE INVENTION

The present invention thus relates to a composition comprising Tr1 cells and mesenchymal stem cells.

In an embodiment of the invention, said composition further comprises the antigen for which the Tr1 cells are specific. In an embodiment of the invention, Tr1 cells are specific for an antigen normally tolerated in a healthy subject.

In a preferred embodiment of the invention, said antigen normally tolerated in a healthy subject is an allergen, a self-antigen, a food antigen or a microbial antigen. Preferably, said allergen is selected from the group comprising pollen, house-dust mite, feline or rodent allergens, moistures, said self-antigen is selected from the group comprising insulin, myelin protein, heat shock proteins, desmogleins, articular proteins, fragments, variants and mixtures thereof, said food antigen is selected from the group comprising ovalbumin, casein, soya protein, gliadin, fragments, variants and mixtures thereof and said microbial antigen is selected from the group comprising Escherichia coli, Enterobacter aerogenes, Enterobacter cloacae and proteins from commensal bacteria.

In another embodiment of the invention, said Tr1 cells and MSCs are autologous.

In another embodiment of the invention, Tr1 cells and MSCs are packaged separately to be administered sequentially or simultaneously.

The present invention also relates to a medicament comprising the composition as described above.

The present invention also relates to a pharmaceutical composition comprising the composition as described above in combination with one or more pharmaceutically acceptable excipients.

Another object of the invention is a method for inducing antigen-immune specific tolerance in a subject suffering of a disease involving an excessive, dysfunctional or uncontrolled self or non-self T cell mediated immune response, comprising administering to said subject an effective amount of a medicament or a pharmaceutical composition as described above.

In an embodiment of the invention, said disease is an autoimmune disease, an allergic disease or an inflammatory disease.

In a preferred embodiment, said autoimmune disease is selected from the group comprising Wegener's disease, Primary biliary Cirrhosis, Primary sclerosing cholangitis, Crohn's disease, rheumatoid arthritis, multiple sclerosis and Insulin resistant diabetes.

In a preferred embodiment, said allergic disease is selected from the group comprising asthma, rhinitis, urticaria, atopic dermatitis; fibrotic diseases and food allergy.

In a preferred embodiment, said inflammatory disease is selected from the group comprising rheumatoid arthritis, multiple sclerosis, Crohn's disease.

In an embodiment of the invention, said Tr1 cells and said MSCs are administrated simultaneously or sequentially.

Another object of the invention is to provide a method for promoting tissue regeneration in a subject in need thereof, comprising administering to said subject an effective amount of the medicament or the pharmaceutical composition of the invention.

Another object of the invention is to provide a method for treating fibrosis in a subject in need thereof, comprising administering to said subject an effective amount of the medicament or the pharmaceutical composition of the invention.

Another object of the invention is to provide a method for promoting angiogenesis in an organ or a tissue of a subject in need thereof, comprising administering to said subject an effective amount of the medicament or the pharmaceutical composition of the invention.

### DETAILED DESCRIPTION OF THE INVENTION

### Definition

The term **"regulatory T cells"** or **"T suppressor"** as used herein refers to a T cell population that inhibits or prevents the activation, or in another embodiment, the effector function and proliferation, of another T lymphocyte.

The term **"Tr1 cells"** as used herein refers to cells having the following phenotype at rest CD4+CD25-FoxP3- and capable of secreting high levels of IL-10 and low to moderate levels of TGF-β upon activation. Tr1 cells are characterized, in part, by their unique cytokine profile: they produce high levels of IL-10, significant levels of TGF-β and intermediate levels of IFN-γ, but little or no IL-4 or IL-2. The cytokine production is typically evaluated in cultures of cells after activation with polyclonal activators of T lymphocytes such as anti-CD3+ anti-CD28 antibodies or Interleukin-2, PMA + ionomycin. Alternatively, the cytokine production is evaluated in cultures of cells after activation with the specific T-cell antigen presented by antigen presenting cells. High levels of IL-10 correspond to at least about 500 pg/ml, typically greater than about 1, 2, 4, 6, 8, 10, 12, 14, 16, 18, or 20 thousand pg/ml or more. Significant levels of TGF-β correspond to at least about 100 pg/ml, typically greater than about 200, 300, 400, 600, 800, or 1000 pg/ml or more. Intermediate levels of IFN-γ correspond to concentrations comprised between 0 pg/ml and at least 400 pg/ml, typically greater than about 600, 800, 1000, 1200, 1400, 1600, 1800, or 2000 pg/ml or more. Little or no IL-4 or IL-2 corresponds to less than about 500 pg/ml, preferably less than about 250, 100, 75, or 50 pg/ml, or less.

The term **"antigen"** as used herein refers to a protein, or peptide, associated with a particular disease for which the cells of this invention are being used to modulate, or for use in any of the methods of this invention. In one embodiment, the term "antigen" may refer to a synthetically derived molecule, or a naturally derived molecule, which shares sequence homology with an antigen of interest, or structural homology with an antigen of interest, or a combination thereof. In one embodiment, the antigen may be a mimetope.

The term **"antigen specific"** as used herein refers to a property of the population of cells such that supply of a particular antigen, or a fragment of this antigen, results in one embodiment in specific regulatory T cells proliferation when presented the antigen in the context of MHC. In another embodiment, supply of the antigen or fragment thereof, results in regulatory T cells production of IL-10. In one embodiment, the regulatory T cell population expresses a monoclonal T cell receptor. In another embodiment, the regulatory T cell population expresses polyclonal T cell receptors.

The term "**self-antigen**" as used herein refers to an antigen that is normally expressed in the body of the subject. In one embodiment, self-antigen refers to an antigen, which when expressed in a body, may result in the education of self-reactive T cells. In one embodiment, self-antigen is expressed in an organ that is the target of an autoimmune disease. In one embodiment, the self-antigen is expressed in a pancreas, thyroid, connective tissue, kidney, lung, liver, digestive system or nervous system. In another embodiment, self-antigen is expressed on pancreatic β cells.

The term "**antigen normally tolerated in a healthy subject**" refers to all self or non-self molecules or entities that did not induce pro-inflammatory response in healthy subjects. These tolerated antigens can be self antigens, ingested antigens, inhaled antigens, bacterial flora antigens or contact antigens.

The term **"subject"** as used herein refers to a mammal, in particular a human being.

The term **"effective amount"** as used herein refers to an amount sufficient to cause a beneficial or desired clinical result (e.g. improvement in clinical condition).

The term **"treatment"** or **"treating"** as used herein generally refers to a clinical intervention in an attempt to alter the natural course of the individual or cell being treated, and may be performed either for prophylaxis or during the course of clinical pathology. Desirable effects include, but are not limited to, preventing occurrence or recurrence of disease, alleviating symptoms, suppressing, diminishing or inhibiting any direct or indirect pathological consequences of the disease, preventing metastasis, lowering the rate of disease progression, ameliorating or palliating the disease state, and causing remission or improved prognosis.

The term "**autoimmune disease**" as used herein refers to an immune response directed against a self-antigen.

The term "**inflammatory condition**" or "**inflammatory disorder"** as used herein refers to any disorder that is, in one embodiment, caused by an "inflammatory response" also referred to, in another embodiment, as "inflammation" or, in another embodiment, whose symptoms include inflammation. By way of example, an inflammatory disorder caused by inflammation may be a septic shock, and an inflammatory disorder whose symptoms include inflammation may be rheumatoid arthritis.

The term **"allergic response"** as used herein refers to an immune system attack against a generally harmless, innocuous antigen or allergen. Allergies may in one embodiment include, but are not limited to, hay fever, asthma, atopic eczema as well as allergies to poison oak and ivy, house dust mites, bee venom, nuts, shellfish, penicillin or other medications, or any other compound or compounds which induce an allergic response.

### The present invention

The present invention relates to a composition comprising Tr1 cells and mesenchymal stem cells (MSCs).

In one embodiment of the invention, MSCs may be obtained by harvesting a MSCs-containing tissue and isolating and expanding said MSCs.

The MSCs obtained may be a homogeneous population or may be a mixed cell population enriched in MSCs. Homogeneous MSCs may be obtained by culturing adherent marrow or periosteal cells, or stroma-vascular fraction of adipose tissue and the MSCs may be identified by specific cell surface markers. The homogeneous MSC compositions are obtained by positive selection of adherent marrow, stroma-vascular fraction of adipose tissue or periosteal cells which are free of markers associated with either hematopoietic cell or differentiated mesenchymal cells. These isolated mesenchymal cell populations display epitopic characteristics associated with only mesenchymal stem cells, have the ability to regenerate in culture without differentiating, and have the ability to differentiate into specific mesenchymal lineages when either induced in vitro or placed in vivo at the site of damaged tissue. In order to obtain subject human mesenchymal stem cells, it is necessary to isolate rare pluripotent mesenchymal stem cells from other cells in the bone marrow or other MSC source. Bone marrow cells may be obtained from iliac crest, femora, tibiae, spine, rib or other medullary spaces. Other sources of human mesenchymal stem cells include embryonic yolk sac, placenta, umbilical cord, fetal and adolescent skin, blood and adipose tissues.

A method, incorporated herewith by reference, for obtaining a cell population enriched in MSCs is described for example in the patent US5486359.

In one embodiment of the invention, Tr1 cells may be obtained by
a) isolating a progenitor cell population from a subject;
b) obtaining a population of dendritic cells by culturing said progenitor cell population in presence of IL-10;
c) contacting cells of step b) with a CD4+ T lymphocyte population isolated from said subject in the presence of an antigen to allow differentiation of said CD4+ T cells into the Tr1 cell population, and;
d) recovering the Tr1 cell population from the step c).

In step b), IL-10 is present from 50 to 250 U/ml, preferably at 100 U/ml in the culture medium. Said method for obtaining Tr1 cells is described in Wakkach et al (Immunity 2003 May; 18(5):605-17), incorporated herewith by reference.

Said method may also be carried out using Dexamethasone, Vitamin D3, or tolerogenised or immature DCs instead of the DCs of step b).

In another embodiment of the present invention, Tr1 cells may be obtained by:
a) culturing a CD4+ T cell population isolated from a subject in a media with an appropriate amount of IFN-α, and;
b) recovering the Tr1 cell population.

IFN-α is preferably present in the media at 5 ng/ml. In the step a), the media may further comprise an appropriate amount of IL-10, preferably at 100 U/ml.

In step b), the Tr1 cell population is cultured in a media comprising IL-15 to allow proliferation, IL-15 being preferably at 5 ng/ml in the media. Said method, incorporated herewith by reference, for obtaining Tr1 cells is described in the patent US6746670.

In still another embodiment of the invention, Tr1 cells may be obtained by:
a) in vitro activating a CD4+ T cell population in presence of the antigen, presented by artificial antigen presenting cells, and;
b) recovering an activated CD4+ T cells comprising at least 10% of Tr1 cells.

Preferably, the artificial antigen presenting cells express a HLA II system molecule and a human LFA-3 molecule and don't express the co-stimulation molecules B7-1, B7-2, B7-H1, CD40, CD23 and ICAM-1.

Said process, incorporated herewith by reference, for obtaining Tr1 cells is described in the patent application WO02/092793.

In still another embodiment of the invention, Tr1 cells may be obtained by:
a) in vitro activating a CD4+ T cell population in presence of an antigen and an appropriate amount of IL-10; and;
b) recovering the Tr1 cell population.

Preferably, IL-10 is present in the media at 100 U/ml. Said method is described in Groux et al. (Nature 1997, 389(6652):737-42), incorporated herewith by reference.

In still another embodiment of the invention, antigen-specific Tr1 cells may be obtained by:
a) stimulating a leukocyte population or a peripheral blood mononuclear cell (PBMC) population with an antigen;
b) recovering the antigen-specific Tr1 cell population from the stimulated population;
c) optionally expanding said antigen-specific Tr1 cell population.

Leukocytes encompass several types of cells, which are characterized by their importance, their distribution, their number, their lifetime and their potentiality. These types are the following: the polynuclear or granular leukocytes, among which one finds the eosinophilic, the neutrophilic and the basophilic leukocytes, and the mononuclear cells, or peripheral blood mononuclear cells (PBMCs), which are large white blood cells and consist in the cell types of the immune system (lymphocytes and monocytes). The leukocytes or the PBMCs can be separated from the peripheral blood by any method known to those skilled in the art. Advantageously, for the separation of the PBMCs, centrifugation may be used, preferably density gradient centrifugation, preferably discontinuous density gradient centrifugation. An alternative is the use of specific monoclonal antibodies. In certain embodiments PBMC are typically isolated from the whole blood product by means of Ficoll-Hypaque, using standard procedures. In other embodiments the PBMCs are recovered by means of leukapheresis.

Said method, incorporated herewith by reference, is described in the patent application WO2007/010406.

In still another embodiment, Tr1 cells may be obtained by:
a) culturing a leukocyte population or a peripheral blood mononuclear cell (PBMC) population with mesenchymal stem cells in the presence of an antigen;
b) recovering the Tr1 cell population.

Said method can also be carried out with naive or memory T cells instead of PBMC or leukocytes.

The Tr1 cell population thus obtained may further be expanded by culture in presence of cytokines such as Interleukine-2 and Interleukine-4. Alternatively, Interleukine-15 and Interleukine-13 could also be used in Tr1 cell expansion cultures.

In still another embodiment, Tr1 cells may be obtained by:
a) culturing CD4+ T cells with dendritic cells previously treated with dexamethasone (about 10⁻⁷ M) and a selected antigen;
b) recovering the Tr1 cell population one week after the beginning of the culture.

In the methods described above, Tr1 cells can be characterized by the identification method described in WO2005/000344. Said identification method of Tr1 cells is based on the detection of the simultaneous presence of expression products of genes coding CD4 molecule and molecules from the group comprising CD18 and/or CD11a, and CD49b. Tr1 cells can be identified and/or purified by Elisa, flow cytometry, or immunoaffinity purification methods using antibodies directed against said markers.

Tr1 cells can also be enriched by positive selection or negative selection using flow cytometry or magnetic beads. Such methods, incorporated herewith by reference, are also described in WO2005/000344.

In another embodiment of the invention, said composition comprising Tr1 cells and MSCs can be obtained by co-culture of MSCs with autologous T cells during one to two weeks.

In a preferred embodiment of the invention, the composition comprising said Tr1 cells and said MSCs, further comprises the antigen for which the Tr1 cells are specific.

In one embodiment of the invention, the antigen for which the Tr1 cells are specific may be administrated separately to the composition of the invention, for example a dietary antigen may be administrated in food to a subject. In another embodiment, the antigen for which the Tr1 cells are specific is added in the composition of the invention.

In a preferred embodiment of the invention, said antigen for which the Tr1 cells are specific is an antigen normally tolerated in a healthy subject.

In one embodiment of the invention, said antigen normally tolerated in a healthy subject is an allergen. A list of allergen can be found on http://www.allergen.org/.

On a preferred embodiment, said allergen is selected from the group of pollen, house-dust mite, feline or rodent allergens, moistures.

In another embodiment of the invention, said antigen normally tolerated in a healthy subject is a food antigen.

The term "food-antigen" refers to an immunogenic peptide, which comes from foodstuffs, such as food antigens of the following non-limiting list: bovine antigens such as lipocalin, Ca-binding SlOO, alpha-lactalbumin, beta-lactoglobulin, bovine serum albumin, immunoglobulin or caseins. Food-antigens may also be atlantic salmon antigens such as parvalbumin, chicken antigens such as ovomucoid, ovalbumin, Ag22, conalbumin, lysozyme or chicken serum albumin, peanuts, shrimp antigens such as tropomyosin, wheat antigens such as agglutinin or omega-5 gliadin, celery antigens such as celery profilin, carrot antigens such as carrot profilin, apple antigens such as thaumatin, apple lipid transfer protein, apple profilin, pear antigens such as pear profilin, isoflavone reductase, avocado antigens such as endochitinase, apricot antigens such as apricot lipid transfer protein, peach antigens such as peach lipid transfer protein or peach profilin, soybean antigens such as HPS, soybean profilin or (SAM22) PR-10 prot.

In another embodiment of the invention, said antigen normally tolerated in a healthy subject is a self- antigen.

The term "self-antigen" refers to an immunogenic peptide derived from a protein of said individual. It may be, by way of example, an auto-antigen of the following non-limiting list: acetylcholine receptor, actin, adenin nucleotide translocator, β- adrenoreceptor, aromatic L-amino acid decarboxylase, asioaloglycoprotein receptor, bactericidal/permeability increasing protein (BPi), calcium sensing receptor, cholesterol side chain cleavage enzyme, collagen type IV Oy-chain, cytochrome P450 2D6, desmin, desmoglein-1, desmoglein-3, F-actin, GM- gangliosides, glutamate decarboxylase, glutamate receptor, H/K ATPase, 17-[alpha]- hydroxylase, 21 -hydroxylase, IA-2 (ICAS 12), insulin, insulin receptor, intrinsic factor type 1, leucocyte function antigen 1, myelin associated glycoprotein, myelin basic protein, myelin oligodendrocyte protein, myosin, P80-coilin, pyruvate deshydrogenase complex E2 (PDC-E2), sodium iodide symporter, SOX-10, thyroid and eye muscle shared protein, thyroglobulin, thyroid peroxydase, thyrotropin receptor, tissue transglutaminase, transcription coactivator p75, tiyptophan hydroxylase, tyrosinase, tyrosine hydroxylase, ACTH, aminoacyl- tPvNA-hystidyl synthetase, cardiolipin, carbonic anhydrase II, cebtromere associated proteins, DNA-dependant nucleosome-stimulated ATPase, fibrillarin, fibronectin, glucose 6 phosphate isomerase, beta 2-glycoprotein I, golgin (95, 97, 160, 180), heat shock proteins, hemidesmosomal protein 180, histone H2A, H2B, keratin, IgE receptor, Ku-DNA protein kinase, Ku-nucleoprotein, La phosphoprotein, myeloperoxydase, proteinase 3, RNA polymerase I-III, signal recognition protein, topoisomerase I, tubulin, vimenscin, myelin associated oligodendrocyte basic protein (MOBP), proteolipid protein, oligodendrocyte specific protein (OSP/Claudin 11), cyclic nucleotide 3'-phosphodiesterase (CNPase), BP antigen 1 (BPAGl-e), transaldolase (TAL), human mitochondrial autoantigens PDC-E2 (Novo 1 and 2), OGDC-E2 (Novo 3), and BCOADC-E2 (Novo 4), bullous pemphigoid (BP)180, laminin 5 (LN5), DEAD-box protein 48 (DDX48) or insulinoma-associated antigen-2.

Preferably, the food- or self-antigen is a recombinant or a synthesized antigen.

Preferably, the antigen is a food-antigen selected from the group comprising ovalbumin, casein, soya protein, gliadin, peanuts, fragments, variants and mixtures thereof.

Preferably, the antigen is a self-antigen selected from the group comprising insulin, myelin protein, heat shock proteins, desmogleins, articular proteins, proteinase 3, fragments, variants and mixtures thereof.

The term "variant" of the food- or auto-antigen refers herein to an antigen that is almost identical to the natural antigen and which shares the same biological activity. The minimal difference between the natural antigen and its variant may lie for example in an amino-acid substitution, deletion, and/or addition. Such variants may contain for example conservative amino acid substitutions in which amino acid residues are replaced with amino acid residues having a similar side chain. Families of amino acid residues having similar side chains have been defined in the art, including basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta. -branched side chains (e.g., threonine, valine, isoleucine) and aromatic side chains (e.g., tyrosine, phenylalanine, tryptophan, histidine).

In one embodiment of the invention, said antigen normally tolerated in a healthy subject is a microbial antigen.

Microbial antigen includes, but is not limited to, antigen derived from a microorganism such as a bacterium, archaebacterium, fungus, virus, protozoan, parasite, alga, slime mold, or prion.

Examples of said microorganisms are Streptococcus pneumoniae, Staphylococcusaureus, Clostridium difficile, Haemophilus influenza, Pseudomonas aeruginosa, Neisseria meningitidis, Escherichia coli, Helicobacter pylori, Moraxella catarrhalis, Mycobacteria, Salmonella, Vibrio, Streptomyces, Helicobacter, Lactococcus and Listeria.

Preferably, the antigen is a microbial antigen selected from the group comprising Escherichia coli, Enterobacter aerogenes, Enterobacter cloacae and proteins from commensal bacteria.

In a preferred embodiment of the invention, said Tr1 cells and MSCs are autologous.

This means that MSCs and Tr1 cells or precursors thereof are obtained from the same subject and will be administrated to the subject they come from.

MSCs and Tr1 cells being autologous first allows a long term engraftment: there will be no rejection of the cells and no allogeneic responses. Second, in case an antigen presentation by the MSCs is needed, the autologous context makes it possible.

The present invention also relates to a composition as described above, wherein Tr1 cells and MSCs are packaged separately to be administered sequentially or simultaneously.

By sequentially, it is meant that MSCs may be injected first and Tr1 cells be injected in second, preferably 24 to 48h after MSCs injection.

The present invention also relates to a medicament comprising the composition described above.

The present invention also relates to a pharmaceutical composition comprising the composition as described above in combination with one or more pharmaceutically acceptable excipients.

It is another object of the present invention to provide a method for inducing antigen-immune specific tolerance in a subject suffering of a disease involving an excessive, dysfunctional or uncontrolled self or non-self T cell mediated immune response, comprising administering to said subject an effective amount of a medicament or a pharmaceutical composition as described above.

Suitable carriers and diluents include isotonic saline solutions, for example phosphate-buffered saline. The composition may be formulated for parenteral, intramuscular, intravenous, intra-peritoneal, injection, intranasal inhalation, lung inhalation, intradermal, intra-articular, intrathecal, or via the alimentary tract.

Medicament or pharmaceutical compositions of the invention are typically administrated to the patient by intramuscular, intraperitoneal or intravenous injection, or by direct injection into the lymph nodes of the patient, preferably by intravenous injection.

Typically 10⁴/kg to 10⁹/kg cells, preferably 10⁵/kg to 10⁷/kg cells and more preferably about 10⁶/kg cells are administrated to the subject.

The routes of administration and dosages described are intended only as a guide as a skilled practionner will be able to determine readily the optimum route of administration and dosage for any particular subject, depending on for example the age, weight and condition of the patient.

In a preferred embodiment, said disease involving an excessive, dysfunctional or uncontrolled self or non-self T cell mediated immune response is an autoimmune disease, an allergic disease or an inflammatory disease.

Autoimmune diseases include but are not limited to diabetes, multiple sclerosis, and rheumatoid arthritis. Particular conditions associated with autoimmune diseases which may be treated, include: autoimmune (Hasimoto's) thyroiditis, hyperthyroidism (Graves' disease) type I diabetes mellitus, insulin resistant diabetes, autoimmune adrenal insufficiency (Addison's disease), autoimmune oophoritis, autoimmune orchitis, autoimmune hemolytic anemia, paroxysmal cold hemoglobinuria, autoimmune thrombocytopenia, autoimmune neutropenia, pernicius anemia, pure red cell anemia, autoimmune coagulopathies, myasthenia gravis, autoimmune polyneuritis, multiple sclerosis, pemphigus and other bullous diseases, rheumatic carditis, Goodpasture's syndrome, postcardiotomy syndrome, systemic lupus erythematosus, rheumatoid arthritis, Sjorgen's syndrome, polymyositis, dermatomyositis, scleroderma; inflammatory bowel diseases: Crohn's disease, ulcerative colitis; chronic obstructive pulmonary diseases; chronic inflammatory diseases, Coelic disease, Wegener's disease, Primary biliary Cirrhosis, Primary sclerosing cholangitis, Autoimmune hepatitis, Spondylarthritis.

Preferably, said autoimmune disease is selected in the group comprising Wegener's disease, Primary biliary Cirrhosis, Primary sclerosing cholangitis, Crohn's disease, rheumatoid arthritis, multiple sclerosis and Insulin resistant diabetes.

Allergic diseases include, but are not limited to, asthma, rhinitis, urticaria, atopic dermatitis; fibrotic diseases and food allergy.

The inflammatory disorders include but are not limited to cardiovascular disease, rheumatoid arthritis, multiple sclerosis, Crohn's disease, inflammatory bowel disease, systemic lupus erythematosis, polymyositis, septic shock, graft versus host disease, host versus graft disease, asthma, rhinitis, psoriasis, cachexia associated with cancer, or eczema.

Preferably, said inflammatory disease is selected in the group comprising rheumatoid arthritis, multiple sclerosis, Crohn's disease.

In one embodiment of the method of the invention, said tolerated antigen specific Tr1 cells and said MSCs are administrated simultaneously or sequentially.

By sequentially, it is meant that MSCs may be injected first and Tr1 cells be injected in second, preferably 24 to 48h after MSCs injection.

In another embodiment of the method of the invention, the medicament or pharmaceutical composition of this invention may be administrated in combination with traditional therapies, or in another embodiment, with reduced dosages of such traditional therapies. For example, the method of this invention may be accompanied by the administration of immunosuppressants, the dosage of the immunosuppressant or the number of immunosuppressants being reduced.

Another object of the present invention is to provide a method for promoting tissue regeneration in a subject, comprising administering to said subject an effective amount of a medicament or a pharmaceutical composition as described above.

Examples of tissues to be treated include, but are not limited to, muscle, bone and cartilage regeneration.

Suitable carriers and diluents include isotonic saline solutions, for example phosphate-buffered saline. The composition may be formulated for parenteral, intramuscular, intravenous, intra-peritoneal, injection, intranasal inhalation, lung inhalation, intradermal, intra-articular, intrathecal, or via the alimentary tract (for example via the Peyers patches). Preferably, the medicament or pharmaceutical composition of the invention may be administrated directly to a degenerated tissue.

Typically 10⁴/kg to 10⁹/kg cells, preferably 10⁵/kg to 10⁷/kg cells and more preferably about 10⁶/kg cells are administrated to the subject.

The routes of administration and dosages described are intended only as a guide as a skilled practionner will be able to determine readily the optimum route of administration and dosage for any particular subject, depending on for example the age, weight and condition of the patient, and the extend and severity of the wound being treated.

Another object of the present invention is to provide a method for treating fibrosis in a subject, comprising administering to said subject an effective amount of a medicament or a pharmaceutical composition as described above.

Examples of fibrosis to be treated include, but are not limited to, cirrhosis of the liver, fibrosis of the kidneys associated with and-stage renal disease, and fibrosis of the lung.

Suitable carriers and diluents include isotonic saline solutions, for example phosphate-buffered saline. The composition may be formulated for parenteral, intramuscular, intravenous, intra-peritoneal, injection, intranasal inhalation, lung inhalation, intradermal, intra-articular, intrathecal, or via the alimentary tract.

Medicament or pharmaceutical composition of the invention are typically administrated to the patient by intramuscular, intraperitoneal or intravenous injection, or by direct injection into the lymph nodes of the patient, preferably by direct intravenous injection.

Typically 10⁴/kg to 10⁹/kg cells, preferably 10⁵/kg to 10⁷/kg cells and more preferably about 10⁶/kg cells are administrated to the subject.

The routes of administration and dosages described are intended only as a guide as a skilled practionner will be able to determine readily the optimum route of administration and dosage for any particular subject, depending on for example the age, weight and condition of the subject, and the extent and severity of the fibrosis being treated.

Another object of the present invention to provide a method for promoting angiogenesis in a tissue or organ in a subject wherein such tissue or organ is in need of angiogenesis, comprising administering to said subject an effective amount of a medicament or a pharmaceutical composition as described above.

The induction of angiogenesis may be used to treat coronary and peripheral artery insufficiency, and thus may be a non invasive and curative approach to the treatment of coronary artery disease, ischemic heart disease, and peripheral artery disease. Angiogenesis may play a role in the treatment of diseases and disorders in tissues and organs other than the heart, as well as in the development and/or maintenance of organs other than the heart. Angiogenesis may provide a role in the treatment of internal and external wounds, as well as dermal ulcers. Angiogenesis is also essential for the coupling of cartilage resorption with bone formation, and is essential for correct growth plate morphogenesis. Angiogenesis also plays a role in embryo implantation, and placental growth, as well as in the development of the embryonic vasculature.

Suitable carriers and diluents include isotonic saline solutions, for example phosphate-buffered saline. The composition may be formulated for parenteral, intramuscular, intravenous, intra-peritoneal, injection, intranasal inhalation, lung inhalation, intradermal, intra-articular, intrathecal, or via the alimentary tract.

Medicament or pharmaceutical composition of the invention are typically administrated to the patient by intramuscular, intraperitoneal or intravenous injection, or by direct injection into the lymph nodes of the patient, preferably by direct intravenous injection.

Typically 10⁴/kg to 10⁹/kg cells, preferably 10⁵/kg to 10⁷/kg cells and more preferably about 10⁶/kg cells are administrated to the subject.

The routes of administration and dosages described are intended only as a guide as a skilled practionner will be able to determine readily the optimum route of administration and dosage for any particular subject, depending on for example the age, weight and condition of the patient.

### BRIEF DESCRIPTION OF THE FIGURES

**Figure 1****:** detection of IFN-gamma produced by T cells activated in the presence or absence of MSCs and/or Tr1 cells.
   MLR was cultured in the lower well of a transwell alone or in presence of MSCs, Tr1 or MSCs and Tr1 in the upper well. Supernatant was collected after 4 days and IFNγ secretion was measured by ELISA.
**Figure 2****:** MSCs co-administration improves the efficacy of Tr1 cell therapy in a model of colitis in mice.

Balb/c mice were treated with DSS in the drinking water for 7 days, Tr1 cells and MSCs were injected intravenously the first day. Clinical score and body weight were monitored daily.

### EXAMPLES

### Example 1

### Preparation of MSCs

Mesenchymal stem cells were derived from the stroma-vascular fraction of the adipose tissue of Balb/c mice. Adipose tissue was digested with 0,1% collagenase for 30 minutes and filtered through a 70µ mesh. Adherent cells were cultured in RPMI medium containing 10% FCS and 10% horse serum supplemented with glutamine and penicillin and streptomycin. Frequently, cells were monitored for their capacity to differentiate in both adipocytes and osteoblasts lineages.

### Preparation of Tr1 cells

Ovalbumin specific Tr1 cells were differentiated from naïve CD4+ T lymphocytes from DO11-10 ovalbumin specific TCR transgenic mice after activation with ovalbumin peptide 323-339 and IL-10 in the presence of irradiated syngeneic antigen presenting cells. Cells were then clones by limiting dilution in order to obtain monoclonal population of ovalbumin specific Tr1 cells. Cells used in experiment 1 and 2 were derived from the culture of a Tr1 clone.

### Activation assay of T cells

Suppressive capacity of Tr1 cells and MSCs was evaluated on the inhibition of the MLR. MLRs were set up in the lower well of transwell with 10⁶ responder cells (Balb/c mice) and 10⁶ irradiated splenocytes from C57 BL6 mice. MSCs (3x10⁴ cells) and Tr1 cells (10⁵ cells) were added in the upper well. After 4 days, supernatant of the MLR was collected and IFNgamma secretion was measured by ELISA.

### Method for determining IFNgamma production

Interferon gamma production by activated T lymphocytes was evaluated by commercially available ELISA purchased from BD Biosciences.

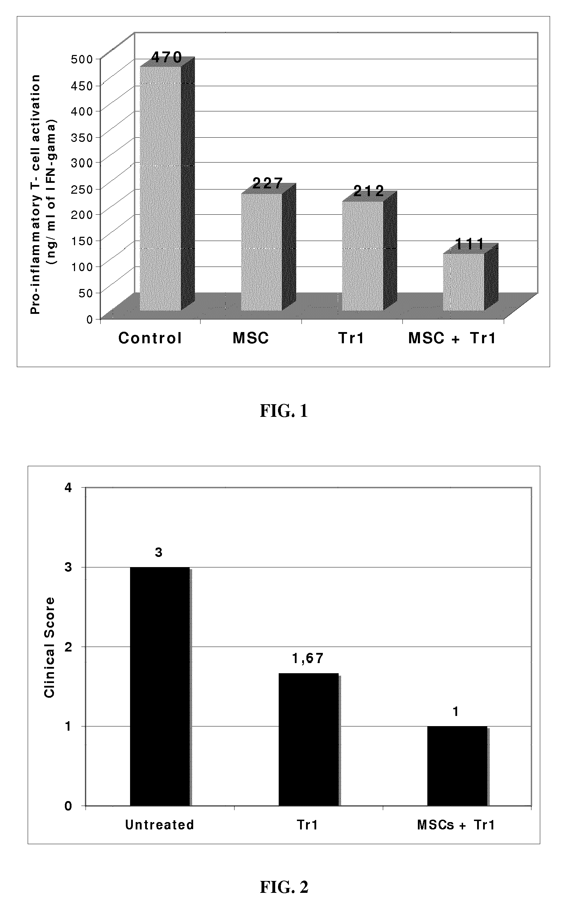

### Results observed (figure 1)

Results demonstrate that both MSC and Tr1 cells independently inhibits T-cell activation measured by the diminution of IFNgamma released by pro-inflammatory T lymphocytes. Co-culture of MSC and Tr1 cells allows a greater inhibition compared to MSC or Tr1 cells alone showing that the two type of cells displays a synergism of action that leads to enhanced inhibition of T-cell activation and IFNgamma release.

### Example 2

BALB/c mice were treated with Dextran Sodium Sulfate (DSS, 5% in drinking water) to induce acute colitis. Group of mice were left untreated or treated intravenously at day 1 with 10⁶ ovalbumin specific Tr1 cells with or without adipose tissue derived MSCs (0.5x10⁶/mouse). After 7 Days, clinical signs of mice were evaluated based on the following scoring:
- 0-: No clinical signs;
- 1-: Weight loss;
- 2-: Weight loss + mild Diarrhea;
- 3-: Weight loss + severe Diarrhea;
- 4-: Weight loss + severe Diarrhea + blood in the feces.

Results show that MSCs co-administration improves the efficacy of Tr1 cell therapy in this model of colitis (figure 2).

## Claims

1. Composition comprising Tr1 cells and mesenchymal stem cells.

2. Composition according to claim **1,** further comprising the antigen for which the Tr1 cells are specific.

3. Composition according to claim **1** or **2,** wherein Tr1 cells are specific for an antigen normally tolerated in a healthy subject.

4. Composition according to claim **1** or **2,** wherein said antigen normally tolerated in a healthy subject is an allergen, a self-antigen, a food antigen or a microbial antigen.

5. Composition according to anyone of claims **1** to **4,** wherein said Tr1 cells and MSCs are autologous.

6. Composition according to anyone of claims **1** to **5,** wherein Tr1 cells and MSCs are packaged separately to be administered sequentially or simultaneously.

7. Medicament comprising a composition according to anyone of claims **1** to **6.**

8. Pharmaceutical composition comprising the composition according to anyone of claims **1** to **6** in combination with one or more pharmaceutically acceptable excipients.

9. Method for inducing antigen-immune specific tolerance in a subject suffering of a disease involving an excessive, dysfunctional or uncontrolled self or non-self T cell mediated immune response, comprising administering to said subject an effective amount of a medicament according to claim **7** or a pharmaceutical composition according to claim **8.**

10. The method according to claim **9** wherein said disease is an autoimmune disease, an allergic disease or an inflammatory disease.

11. The method according to claim **9,** wherein said disease is an autoimmune disease selected from the group comprising Wegener's disease, Primary biliary Cirrhosis, Primary sclerosing cholangitis, Crohn's disease, rheumatoid arthritis, multiple sclerosis and Insulin resistant diabetes.

12. The method according to claim **9,** wherein said disease is an inflammatory disease selected from the group comprising rheumatoid arthritis, multiple sclerosis, Crohn's disease.

13. The method according to claim **9,** wherein said disease is an allergic disease selected from the group comprising asthma, rhinitis, urticaria, atopic dermatitis; fibrotic diseases and food allergy.

14. The method according to anyone of claims **9** to **13,** wherein said Tr1 cells and said MSCs are administrated simultaneously or sequentially.

15. Method for promoting tissue regeneration in a subject in need thereof, comprising administering to said subject an effective amount of a medicament according to claim 7 or a pharmaceutical composition according to claim **8.**

16. Method for treating fibrosis in a subject in need thereof, comprising administering to said subject an effective amount of a medicament according to claim **7** or a pharmaceutical composition according to claim **8.**

17. Method for promoting angiogenesis in an organ or a tissue of a subject in need thereof, comprising administering to said subject an effective amount of a medicament according to claim **7** or a pharmaceutical composition according to claim **8.**
